# EUROPÄISCHE PATENTSCHRIFT

(11) **EP 1 692 256 B1**
(45) Veröffentlichungstag und Bekanntmachung des Hinweises auf die Patenterteilung: **23.01.2013**
(21) Anmeldenummer: 04819643.0
(22) Anmeldetag: 01.12.2004
(51) Int. Cl.: C12M 1/107

(54) **GROSSFERMENTER ZUR ERZEUGUNG VON BIOGAS AUS BIOMASSE**
LARGE-SCALE FERMENTER FOR THE GENERATION OF BIOGAS FROM BIOMASS
FERMENTEUR DE GRANDES DIMENSIONS SERVANT A GENERER UN BIOGAZ A PARTIR D'UNE BIOMASSE

(30) Priorität: 01.12.2003 DE 20318783 U; 23.12.2003 DE 20319847 U
(43) Veröffentlichungstag der Anmeldung: 23.08.2006
(73) Patentinhaber: BEKON Holding AG, 85774 Unterföhring (DE)
(72) Erfinder: Lutz, Peter, 81927 München (DE)
(74) Vertreter: Winter, Brandl, Fürniss, Hübner, Röss, Kaiser, Polte - Partnerschaft
(86) Internationale Anmeldenummer: PCT/EP2004/013646
(87) Internationale Veröffentlichungsnummer: WO 2005/054423

(56) Entgegenhaltungen:
- EP-A- 0 934 998
- WO-A-00/53542
- WO-A-02/06439
- GB-A- 2 072 649
- US-A- 4 643 111
- PATENT ABSTRACTS OF JAPAN Bd. 006, Nr. 191 (C-127), 30. September 1982 (1982-09-30) & JP 57 105296 A (MATSUSHITA ELECTRIC IND CO LTD), 30. Juni 1982 (1982-06-30)

## Beschreibung

Die Erfindung betrifft eine Biogasanlage zur Erzeugung von thermischer, elektrischer und/oder mechanischer Energie aus Biogas.

Bislang hat sich die Biogastechnik hauptsächlich auf die "Nassvergärung" von Gülle und/oder Bioabfällen aus dem kommunalen Bereich konzentriert. Nachwachsende Rohstoffe mit hohen Trockensubstanzgehalten (z.B. Maissilage) oder Festmiste können bei diesen Verfahren nur in begrenztem Umfang beigemischt werden. Die so genannte "Trockenfermentation" erlaubt es, schüttfähige Biomassen aus der Landwirtschaft, aus Bioabfällen und kommunalen Pflegeflächen zu methanisieren, ohne die Materialien in ein pumpfähiges, flüssiges Substrat zu überführen. Es können Biomassen mit bis zu 50% Trockensubstanzanteil vergoren werden. Dieses Trockenfermentations-Verfahren ist beispielsweise in der EP 0 934 998 A2 beschrieben.

Bei der "trockenen" Vergärung wird das zu vergärende Material nicht in eine flüssige Phase eingerührt, wie das zum Beispiel bei der Flüssigvergärung von Bioabfällen der Fall ist. Stattdessen wird das in den Fermenter eingebrachte Gärsubstrat ständig feucht gehalten, indem das Perkolat am Fermenterboden abgezogen und über der Biomasse wieder versprüht wird. So werden optimale Lebensbedingungen für die Bakterien erreicht. Bei der Rezirkulation des Perkolats kann zusätzlich die Temperatur reguliert werden, und es besteht die Möglichkeit, Zusatzstoffe für eine Prozessoptimierung zuzugeben.

Aus der WO 02/06439 ist ein Biorektor bzw. ein Fermenter in Form einer Fertiggarage bekannt, der nach dem Prinzip der Trockenfermentation im sogenannten Batch-Verfahren betrieben wird. Hierbei wird nach einer Animpfung mit bereits vergorenem Material wird das Gärsubstrat mit Radladern in den Fermenter gefüllt. Der garagenförmig aufgebaute Gärbehälter wird mit einem gasdichten Tor verschlossen. Die Biomasse wird unter Luftabschluss vergoren, dabei erfolgt keine weitere Durchmischung und es wird kein zusätzliches Material zugeführt. Das aus dem Gärgut sickernde Perkolat wird über eine Drainagerinne abgezogen, in einem Tank zwischenges peichert und zur Befeuchtung wieder über dem Gärsubstrat versprüht. Der Gärprozess findet im mesophilen Temperaturbereich bei 34-37 °C statt, die Temperierung erfolgt mittels einer Boden- und Wandheizung. Zur Vermeidung eines explosiven Gasgemisches im Fermenter bei einer befürchteten Leckage kann Abgas in den Fermenter eingepumpt werden.

Das entstehende Biogas kann in einem Blockheizkraftwerk zur Gewinnung von Strom und Wärme genutzt werden. Damit immer genug Biogas für das Blockheizkraftwerk zur Verfügung steht, werden in der Trockenfermentationsanlage mehrere Gärbehälter zeitlich versetzt betrieben. Am Ende der Verweilzeit wird der Fermenterraum vollständig entleert und dann neu befüllt. Das vergorene Substrat wird einer Nachkompostierung zugeführt, so dass ein konventionellen Komposten vergleichbarer organischer Dünger entsteht.

Der aus der WO 02/06439 bekannte Fermenter nach Art einer Fertiggarage läßt sich nicht ohne weiteres vergrößern, falls Anlagen mit größerer Kapazität gewünscht sind. Eine einfache Vergrößerung, z. B. eine Verlängerung der Fertiggaragenfermenter führt zu größeren thermischen Spannungen, die zu Dichtigkeitsproblemen in dem gasdichten Beton führen können.

Aus der GB 2 072 649 A ist ein Naßfermenter zur Erzeugung von Biogas bekannt, bei dem der Fermenterbehälter durch eine Gasdichte Folie abgedeckt ist.

Aus der DE 699 02 902 T2 und aus der DE 200 22 758 U1 sind Biofermenter für flüssige Gärsubstrate bekannt, bei denen die Oberseite des Faulbeälters mit einer flexiblen Folie verschlossen ist. Um die notwendige gasdichte Verbindung zwischen Abdichtfolie und Faulbehälter herzustellen, wird diese Abdichtfolie unter dem Flüssigkeitsniveau der Gärsubstrate an dem Faulbehälter befestigt. Für die Methanisierung von Feststoffen ist diese Konstruktion nicht geeignet, da insbesondere beim Be- und Entladen des Faulbehälters eine Beschädigung der Folie nicht ausgeschlossen werden kann.

Aus der bereits genannten EP 0 934 998 A2 und aus der FR 2 536 158 A2 ist ein Trockenfermenter bekannt, bei dem der Fermenterkörper allseitig mit Folie gasdichter Folie umhüllt wird. Auch hierbei besteht die Gefahr der Beschädigung der Folie beim Wechsel des Fermenterkörpers.

Ausgehend von dem aus der WO 02/06439 bekannten Fermenter ist es Aufgabe der vorliegenden Erfindung eine Biogasanlage anzugeben, die eine erhöhte Betriebssicherheit ermöglicht.

Die Lösung dieser Aufgabe erfolgt durch die Merkmale des Anspruchs 1.

Die Biogasanlage gemäß Anspruch 1 und 2 zeichnet sich durch eine erhöhte Sicherheit aus. Wenn Biogasfermenter undicht werden, kann sich in dem Fermenter ein leicht entzündliches, explosives Biogas/Sauerstoff-Gemisch bilden. Aufgrund von Funkentladung, Zigaretten oder statischer Elektrizität kann es damit zu schweren Explosionen kommen.

Bei der Biogasanlage gemäß Anspruch 2 wird der Sauerstoffpartialdruck in dem jeweiligen Fermenter gemessen bzw. kontinuierlich überwacht. Übersteigt der Sauerstoffpartialdruck einen bestimmten Wert in dem jeweiligen Fermenter, ist dies ein Anzeichen dafür, daß ein Leck aufgetreten ist und Sauerstoff eindringt. Um diesen gefährlichen Zustand zu verhindern, wird bei Überschreiten eines Schwellwertes für den Sauerstoffpartialdruckes der jeweilige Fermenter von der Biogasleitung abgesperrt. Gleichzeitig wird gekühltes Abgas, d.h. im wesentlichen CO₂, aus dem Biogasverbraucher über über eine Abgasspülleitung in den Fermenter mit dem erhöhten Sauerstoffpartialdruck geführt und ein Spülventil in dem Fermenter geöffnet, so daß die in den Fermenter befindlichen Gase aus dem Fermenter entweichen können und schließlich nahezu ausschließlich Kohlendioxid in dem Fermenter verbleibt. Ist der jeweilige vermutlich lecke Fermenter mit Kohlendioxid bzw. Abgas geflutet, kann er ohne Explosionsgefahr geöffnet werden und anschließend repariert werden.

Gemäß einer vorteilhaften Ausgestaltung der Erfindung ist eine Perkolatkonzentriereinrichtung vorgesehen, die dem aus dem Fermenter abgezogenen Perkolat überschüssige Flüssigkeit entzieht und die für die Vergärung wichtigen Inhaltstoffe des Perkolats aufkonzentriert.

Gemäß einer vorteilhaften Ausgestaltung der Erfindung wird durch die Hülle in Form einer gasdichten Folie die für die Großfermenter notwendige Gasdichtigkeit hergestellt. Die Biomasse wird auf einer Bodenplatte mit einer stabilen Umfassung, die sich in etwa senkrecht in die Höhe erstreckt, gelagert. Die Bodenplatte ist gasdicht ausgeführt, während die stabile seitliche Umfassung nur in dem Bereich gasdicht ausgeführt ist, der nicht von der gasdichten Folie überdeckt wird. Die wesentliche Funktion der seitlichen Umfassung besteht in der Aufnahme der in den Fermenter eingelagerte Biomasse. Zum Be- und Entladen des Fermenters ist eine Be- und Entladeöffnung vorgesehen, die Teil der seitlichen Umfassung ist. Die seitliche Umfassung ist dabei so dimensioniert, d. h. die seitliche Umfassung ist so hoch, dass die Biomasse bei beladenem Zustand des Fermenters nicht mit der Hülle in Berührung kommt, auch nicht beim Be- und Entladen der des Fermenters.

Gemäß einer vorteilhaften Ausgestaltung der Erfindung erstreckt sich die gasdichte Hülle über die Außenseite der stabilen Umfassung bis zur Bodenplatte und ist mit dieser gasdicht verbunden.

Alternativ erstreckt sich die gasdichte Hülle teilweise über die Außenseite der stabilen Umfassung und ist mit der Außenseite der seitlichen Umfassung gasdicht verbunden. Hierbei ist dann der Bereich der stabilen Umfassung unterhalb der Angriffslinie der gasdichten Hülle gasdicht ausgeführt.

Eine weitere Alternative besteht darin, daß die seitliche Umfassung gasdicht ausgeführt ist und eine umlaufende Oberkante umfaßt. Die gasdichte Hülle ist dann gasdicht mit der Oberkante der seitlichen Umfassung verbunden.

Sowohl bei der gasdichten Verbindung der Hülle mit der Bodenplatte, mit der Außenseite der seitlichen Umfassung oder mit der Oberkante der seitlichen Umfassung ist aufgrund der Höhe und Beschaffenheit der stabilen seitlichen Umfassung gewährleistet, dass die Biomasse nicht mit der Folie in Berührung kommt. Dies gilt sowohl für den Betrieb des Fermenters als auch für das Be- und Entladen des Fermenters. Beschädigungen der Folie werden dadurch vermieden. Ein Anschrammen an der stabilen Umfassung beim Be- und Entladen ist unkritisch, da die stabile Umfassung gegenüber mechanischen Beanspruchungen wesentlich unempfindlicher ist als die dünne Folie der gasdichten Hülle.

Gemäß einer bevorzugten Ausführungsform der Erfindung handelt es sich bei der gasdichten Hülle um eine elastische Folie, wodurch zum einen Druckschwankungen des erzeugten Biogases problemlos abgefangen werden können und zusätzlich in gewissem Umfang ein variabler Speicherraum für das erzeugte Biogas bereitgestellt wird.

Gemäß einer weiteren vorteilhaften Ausgestaltung der Erfindung ist die gasdichte Hülle als Doppelfolie mit einem Gasraum zwischen den beiden Folien ausgeführt. Hierdurch erhöht sich die Sicherheit, da ein Leck in einer der beiden Folien noch nicht notwendigerweise zu einem Gasleck führt.

Gemäß einer weiteren vorteilhaften Ausgestaltung der Erfindung ist der Zwischenraum zwischen den beiden Folien mit einem Gas gefüllt, wodurch eine thermische Isolierung des Fermenters von der Umgebung bereitgestellt wird. Als isolierendes Gas kommen insbesondere Luft oder Abgas aus dem BHKW in Frage. In wärmeren Regionen kann der Gasraum in der Doppelfolie zusätzlich als solarer Luftkollektor zum Bereitstellen von trockener Luft genutzt werden.

Gemäß einer weiteren vorteilhaften Ausgestaltung der Erfindung wird der Hohlraum zwischen den beiden Folien der Doppelfolie von Abgas durchströmt, das bei der Verbrennung von Biogas, beispielsweise in einem BHKW entsteht. Dies ist insbesondere in kalten Gebieten vorteilhaft, da damit ein zu großer Wärmeverlust des Fermenters verhindert wird. Ein weiterer Vorteil der Verwendung von Abgas in dem Gasraum der Doppelfolie besteht darin, dass das Abgas inert ist. Sollte es zu einer Beschädigung der inneren oder der äußeren Folie kommen, kann sich kein explosives Umgebungsluft/Gas-Gemisch bilden.

Gemäß einer weiteren vorteilhaften Ausgestaltung der Erfindung wird in den Hohlraum zwischen den beiden Folien der Doppelfolie thermisches Isoliermaterial, z. B. Steinwolle, eingebracht, um den Fermenter thermisch von der Umgebung zu isolieren.

Gemäß einer weiteren vorteilhaften Ausgestaltung der Erfindung schließt die Be- und Entladeöffnung mit der Bodenplatte ab, was sehr vorteilhaft ist, wenn es sich um einen befahrbaren Fermenter handelt.

Gemäß einer weiteren vorteilhaften Ausgestaltung der Erfindung ist der Fermenter nach Art eines Fahrsilos ausgestaltet. Dies ermöglicht eine kostengüstige Herstellung des Fermenters nach bekannten Verfahren und mit allgemein erhältlichen Komponenten. Die vorzugsweise betonierte oder gemauerte seitliche Umfassung zeigt auch die zum Befahren mit Radladern oder dergleichen nötige Robustheit. Ein leichtes Anschrammen beim Be- und Entladen des Fahrsilo-Fermenters ist unkritisch.

Gemäß einer weiteren vorteilhaften Ausgestaltung der Erfindung ist über der seitlichen Umfassung ein Stützgerüst vorgesehen, dass ein Herabfallen der Hülle verhindert. In drucklosem Zustand liegt die Hülle außen auf diesem Stützgerüst auf.

Gemäß einer alternativen Ausführungsform ist über der seitlichen Umfassung eine Außengerüst vorgesehen und die gasdichte Hülle ist innen an diesem Außengerüst befestigt. Hierdurch wird ebenfalls der Kontakt mit der Hülle mit der Biomasse verhindert und zusätzlich bietet das Außengerüst einen gewissen Schutz vor Beschädigung der Hülle.

Gemäß einer weiteren vorteilhaften Ausgestaltung der Erfindung ist die gasdichte Hülle außen zumindest teilweise von einer widerstandsfähigen Schutzhülle umgeben. Hierdurch kann auf einfache Weise eine unbeabsichtigte oder mutwillige Beschädigung der gasdichten Hülle verhindert werden.

Sofern der Gasraum zwischen den beiden Folien der Doppelfolie der gasdichten Hülle mit Abgas aus dem Biogasverbraucher, z.B. einem BHKW, durchströmt wird, wird dieses Abgas zuvor über eine Kühleinrichtung soweit abgekühlt, daß es zu keiner Beschädigung der gasdichten Folie aufgrund hoher Temperaturen kommen kann.

Die übrigen Unteransprüche beziehen sich auf weitere vorteilhafte Ausgestaltungen der Erfindung. Weitere Einzelheiten, Merkmale und Vorteile der Erfindung zeigt die nachfolgende Beschreibung bevorzugter Ausführungsformen anhand der Zeichnungen:
Es zeigt:
   Fig. 1 ein Blockschaltbild einer Biogasanlage gemäß der vorliegenden Erfindung;
   Fig. 2 eine perspektivische Darstellung einer ersten Ausführungsform eines Großfermenters, wie er bei der vorliegenden Erfindung eingesetzt wird;
   Fig. 3 eine Schnittdarstellung durch die Ausführungsform nach Fig. 2;
   Fig. 4 eine perspektivische Darstellung einer Mehrzahl von Großfermentern gemäß Fig. 2 bzw. 3;
   Fig. 5 eine Fig. 3 entsprechende Schnittdarstellung einer zweiten Ausführungsform des Großfermenters;
   Fig. 6 eine Fig. 2 entsprechende perspektivischen Darstellung einer dritten Ausführungsform des Großfermenters;
   Fig. 7 eine schematische Darstellung einer ersten Ausführungsform einer Biogasanlage gemäß der vorliegenden Erfindung; und
   Fig. 8 eine schematische Darstellung einer weiteren Ausführungsform einer Biogasanlage gemäß der vorliegenden Erfindung.

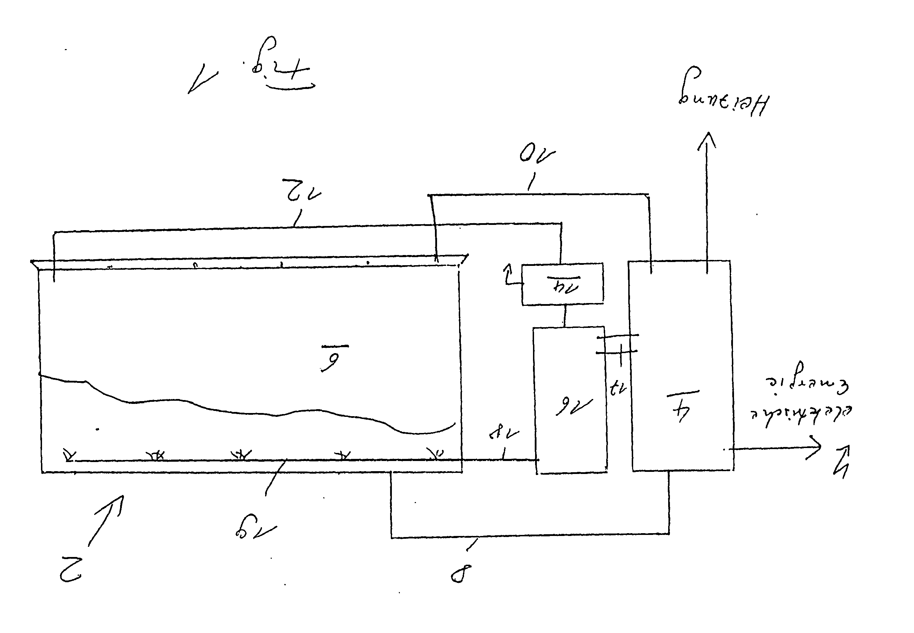

Fig. 1 zeigt eine schematische Darstellung einer herkömmlichen Biogasanlage. Die Biogasanlage umfasst einen Großfermenter 2 in dem Biogas nach dem Trockenfermentations-Verfahren erzeugt wird sowie einen Biogasverbraucher in Form eines Blockheizkraftwerkes (BHKW) 4. Das in dem Großfermenter 2 aus Biomasse 6 erzeugte Biogas wird über eine Biogasentnahmeleitung 8 dem BHKW 4 zugeführt. In dem BHKW 4 wird durch Verbrennung des Biogases elektrische Energie erzeugt und die entstehende Abwärme wird zu Heizzwecken verwendet. Mit der im BHKW 4 entstehenden Wärmeenergie wird der Großfermenter 2 über eine Heizleitung 10 beheizt.

Über eine Perkulatsammelleitung 12 wird das mittels einem nicht näher dargestellten Drainagesystem gesammelte Perkulat aus dem Großfermenter 2 gesammelt und über eine Perkolatkonzentriereinrichtung 14 einem Perkulatsammeltank 16 zugeführt. In der Perkulatkonzentriereinrichtung 14, bei der es sich im Prinzip um eine Filtereinrichtung handelt, wird dem Perkulat überschüssiges Wasser entzogen, so daß die für die Vergärung wichtigen Inhaltsstoffe und Bakterien in höherer Konzentration in dem so behandelten Perkulat vorliegen. Perkulatsammeltank ist über eine Perkulatheizleitung 17 ebenfalls mit dem BHKW 4 verbunden. Hierdurch wird die für die Wirksamkeit des Perkulats optimale Temperatur ermöglicht. Aus dem Perkulatsammeltank 16 führt eine Perkulatverteilerleitung 18 zu einem Perkulatverteiler 19 im Großfermenter 2, um dort das Perkulat von oben über die Biomasse 6 zu versprühen.

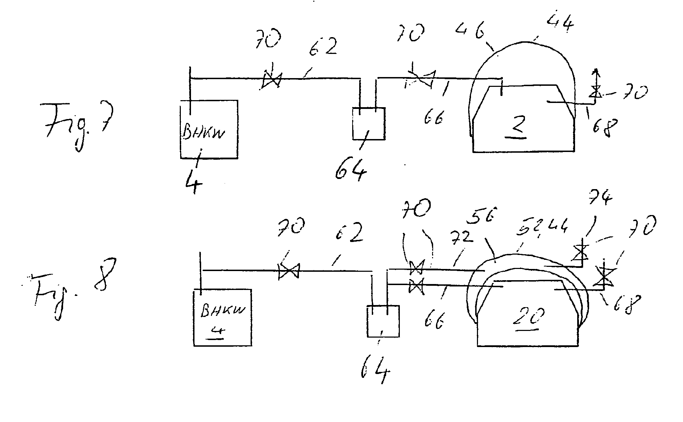

Fig. 7 zeigt schematisch eine erste Ausführungsform der Erfindung mit einem Biogasverbraucher 4 in Form eines BHKW und einem oder mehreren Großfermentern 2 mit einer gasdichten Folie 46 als Hülle 44. Das in dem BHKW 4 durch Verbrennung von Biogas entstehende Abgas wird über eine erste Abgasleitung 62 einem Abgaskühler 64 zugeführt. In dem Abgaskühler 64 wird das heiße Abgas aus dem BHKW 4 beispielsweise durch Einsprühen von Wasser gekühlt. Das gekühlte Abgas kann über eine Abgasspülleitung 66 dem oder den Großfermenter(n) 2 zugeführt werden. Über eine erste Auspuffleitung 68 können die in dem Fermenter enthaltenen Gas ausgeblasen werden. Die Spülung der Fermenter 2 mit gekühltem Abgas erfolgt vor dem Entladen des jeweiligen Fermenters oder im Falle des Auftretens eines Lecks, was zu einem explosiven Biogas-Luft-Gemische in dem jeweiligen Fermenter führen könnte. Die einzelnen Leitungen 62, 66, 68 lassen sich über Ventile 70 absperren. Das Absperren und Öffnen dieser Ventile erfolgt rechnergestützt. Hinsichtlich der konkreten Ausgestaltung Abgaspülung wird auf WO 02/06439 A2 verwiesen.

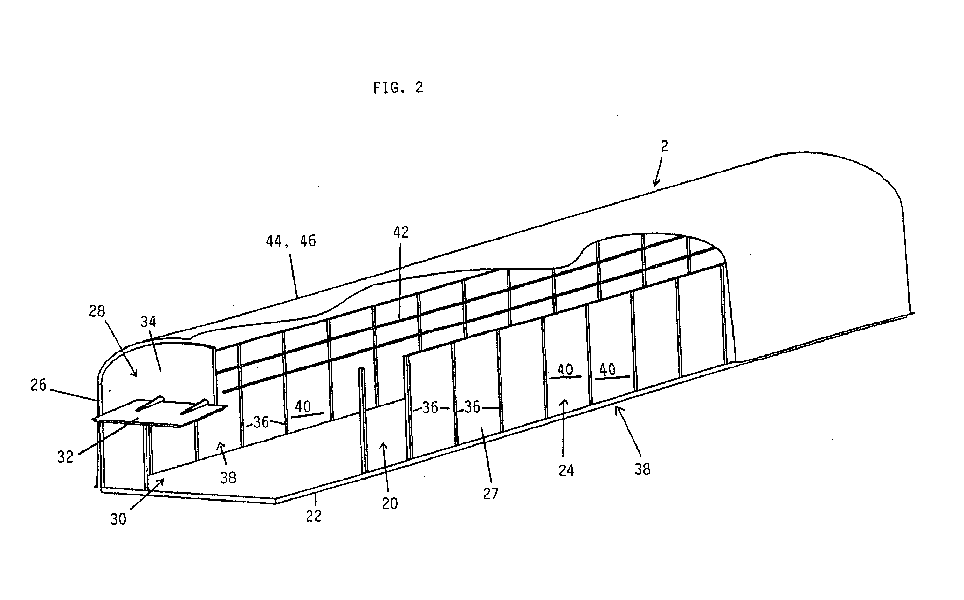

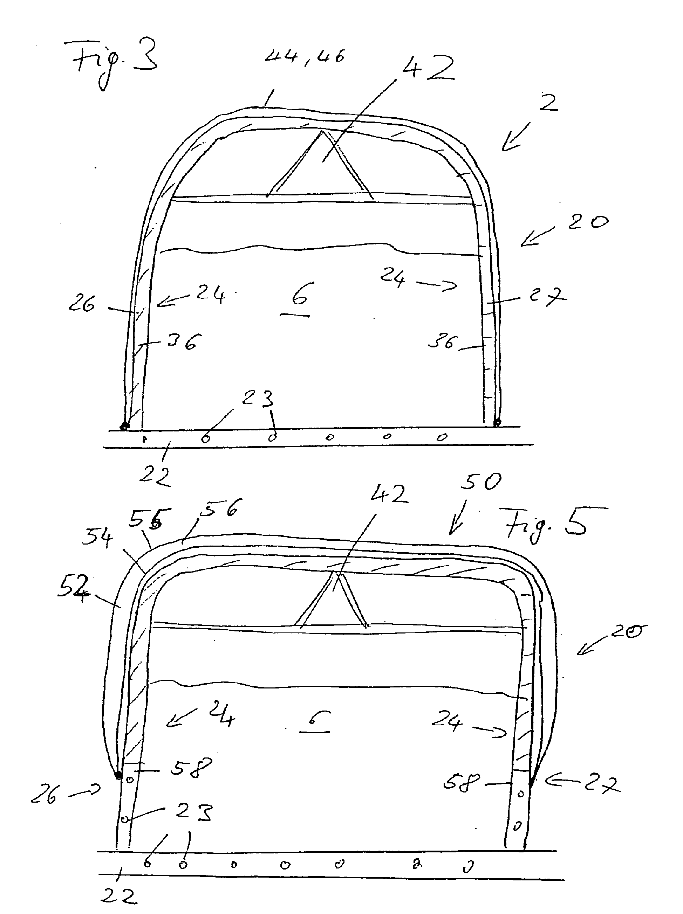

Fig. 2 und 3 zeigen eine erste Ausführungsform des Großfermenters 2, wie er bei der vorliegenden Erfindung eingesetzt werden kann. Bei der ersten Ausführungsform handelt es sich um einen Großfermenter 2 in Form einer langgestreckten überlangen Garage bzw. in Form eines überdachten Fahrsilos. Der Großfermenter 2 umfaßt einen Faulbehälter 20 zur Aufnahme der Biomasse 6. Dieser Faulbehälter 20 umfaßt eine gasdichte Bodenplatte 22 aus Beton auf der eine stabile Umfassung 24 sich nach oben erstreckt und die Biomasse 6 aufnimmt. In der Bodenplate 22 sind nach Art einer Fußbodenheitzung Heizelemente 23 zum Beheizen des Großfermenters 2 vorgesehen. Die stabile Umfassung 24 besteht aus einer Art Fachwerkstruktur mit einer linken Seitenwand 26, einer rechten Seitenwand 27, einer rückwärtigen Abschlußwand - nicht dargestellt - und einer Frontseite 28 mit einer Be- und Entladeöffnung 30, die durch eine gasdichte Klappe 32 abschließbar ist. Die Frontseite 28 und die nicht dargestellte Rückseite bestehen vorzugsweise aus gasdichtem Beton.

Die linke und die rechte Seitenwand 26 und 27 der stabilen Umfassung 24 bestehen aus einer Mehrzahl von senkrechten Säulen 36 in Form von doppel-T-förmigen Trägern mit Zwischenräumen 38. In den Zwischenräumen 38 sind Wandelemente 40 eingefügt, beispielsweise in Form von in die doppel-T-förmigen Säulen 36 eingreifenden Holzplatten oder Holzbretter - nicht näher dargestellt.

Die einzelnen Säulen 36 der linken und rechten Seitenwand 26 und 27 werden über ein Dach- bzw. Stützgerüst 42 miteinander verbunden. Der gesamte Faulbehälter 20 wird durch eine Hülle 44 in Form einer gasdichten Folie 46 gasdicht. Die gasdichte Folie 46 ist gasdicht mit der Bodenplatte 20 verbunden und überdeckt die stabile Umfassung 24 und das Dachgerüst 42. Zum gasdichten Abschluß des Faulbehälters 20 ist die gasdichte Folie 46 auch mit der nicht näher dargestellten Rückfront gasdicht verbunden. Ebenso ist die gasdichte Folie 46 gasdicht mit der Frontseite 28 verbunden. Bei drucklosem Zustand, d. h. wenn der Großfermenter zum Be- und Entladen geöffnet wird, liegt die Hülle 44 auf dem Stützgerüst 42 auf.

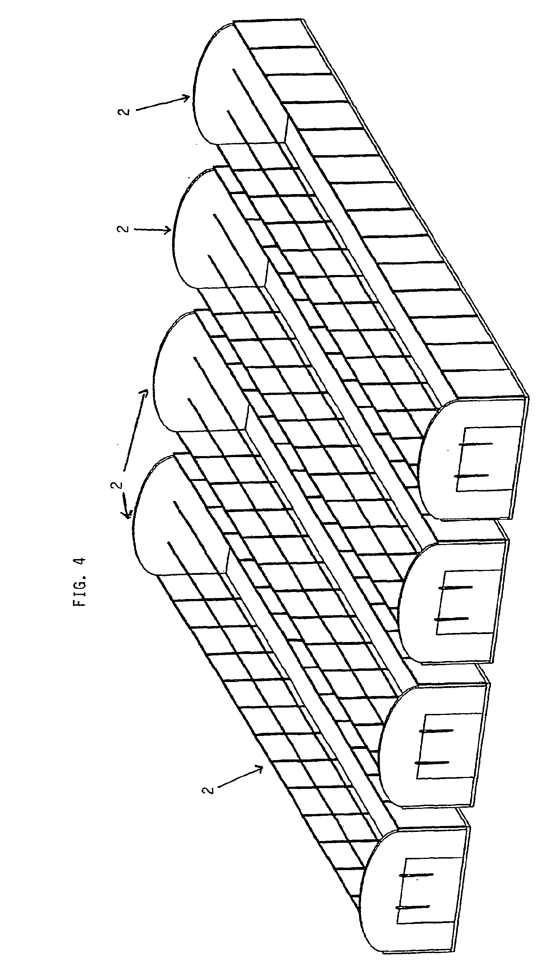

Fig. 4 zeigt eine Mehrzahl der Großfermenter 2 gemäß Fig. 2 bzw. 3 in einer Anordnung nebeneinander. Durch die Anordnung gemäß Fig. 4 wird trotz Batch-Betrieb eine kontinuierliche Gaserzeugung ermöglicht. Hierzu werden die einzelnen Großfermenter 2 zeitlich asynchron mit Biomasse 6 bestückt und entladen.

Fig. 5 zeigt schematische eine zweite Ausführungsform eines Großfermenters 50 in einer Fig. 3 entsprechenden Schnittdarstellung. Der wesentliche Unterschied zur ersten Ausführungsform besteht darin, daß die Hülle 44 aus einer gasdichten Doppelfolie 52 mit einer inneren Folie 54 und einer äußeren Folie 55 mit einem Gasraum 56 zwischen den beiden Folien besteht. Durch Einlagerung eines Gases, z.B. Luft, in diesen Gasraum 56 zwischen den beiden Folien 54 und 55 wird eine thermische Isolierung des Faulbehälters 20 erreicht.

Ein weiterer Unterschied der zweiten Ausführungsform gegenüber der ersten Ausführungsform besteht darin, daß die stabile Umfassung 24 einen gasdichten Sockel 58 umfaßt, der sich von der Bodenplatten 22 nach oben wegerstreckt und zusammen mit der Bodenplatte eine Wanne bildet. Auf der Oberkante des gasdichten Sockels 58 schließt sich dann eine Seitenwandkonstruktion gemäß der ersten Ausführungsform an. Die Doppelfolie 52 ist daher nicht gasdicht mit der Bodenplatte 20, sondern außen am oberen Ende des gasdichten Sockels 58 mit dem Sockel 58 verbunden. Das Vorsehen eines gasdichten Sockels 58 ist vorteilhaft, wenn zusätzlich zu den Heizungselementen 23 in der Bodenplatte 22 noch weitere Heizelemente 23 benötigt werden, die dann in dem gasdichten Sockel 58 angeordnet werden.

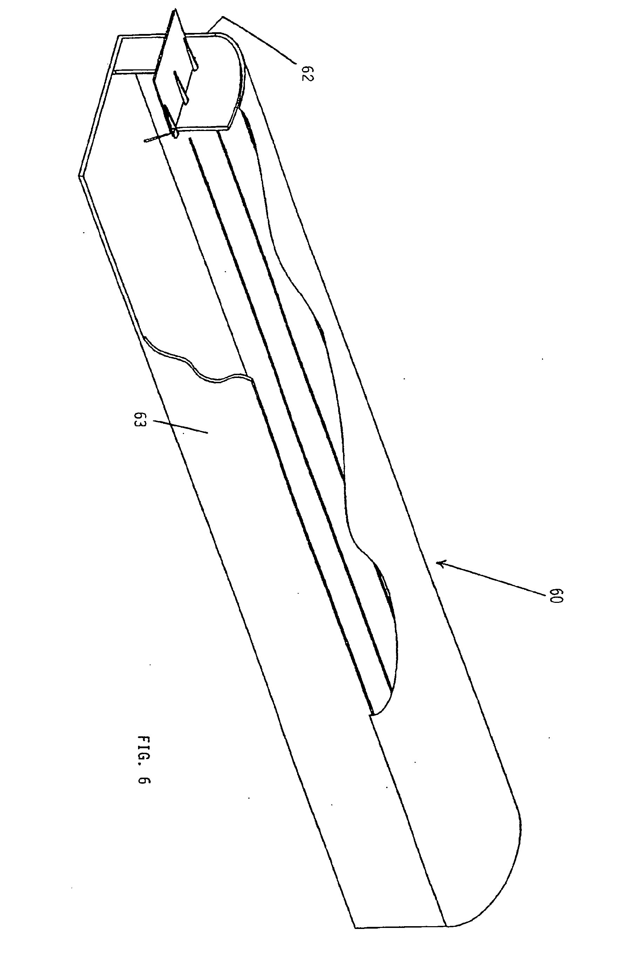

Fig. 6 zeigt eine perspektivische Darstellung einer dritten Ausführungsform eines Großfermenters 60. Diese dritte Ausfürungsform unterscheidet sich von der zweiten Ausführungsform dadurch, das der Sockel 58 aus der zweiten Ausführungsform weiter hochgezogen ist und eine linke und eine rechte Seitenwand 62 und 63 bildet. Die gasdichte Hülle 44 in Form einer einfachen Folie 46 oder in Form der Doppelfolie 52 überspannt bzw. überdeckt lediglich das Dach- bzw. Stützgerüst 42 und ist gasdicht mit der Oberkante der linken und rechten Seitenwand 62 und 63 verbunden. Im übrigen entspricht die dritte Ausführungsform den beiden anderen Ausführungsformen.

Betrieben werden die Großfermenter mittels einem einstufigen Vergärungsverfahren im Batch-Betrieb. "Einstufig" bedeutet hierbei, dass die verschiedenen Abbaureaktionen (Hydrolyse, Säure-, und Methanbildung) zusammen in einem Fermenter ablaufen. Der Begriff "Batch-Betrieb" kennzeichnet ein Verfahrensprinzip, bei dem während des Gärprozesses kein weiteres Material zugeführt oder entnommen wird. Die einmal in den Fermenter gefüllte Biomasse verbleibt dort bis zum Ende der Verweilzeit. Im Gegensatz dazu arbeiten die meisten Flüssigvergärungsverfahren im kontinuierlichen Betrieb, es werden dabei regelmäßig kleinere Mengen Gärsubstrat abgezogen und durch frisches Material ersetzt.

Die Großfermenter werden über eine Boden- und gegebenenfalls über eine Wandheizung temperiert. Auf diese Weise kann die gesamte Kontaktfläche Gärsubstrat/Fermenter zur Wärmeübertragung genutzt werden. Die Heizschleifen werden bereits beim Bau der Behälter in die Betonwände integriert, so dass keine störenden Einbauten im Behälterinneren nötig sind. Zusätzlich kann über die Perkolatheizleitung 17 und einen Wärmetauscher das zugeführte Perkolat temperiert werden. So ist eine optimale Steuerung der Temperatur im Fermenter gewährleistet.

Die Großfermenter bzw. Faulbehälter werden mit hydraulisch betriebenen, gasdichten Toren, den Klappen 32 verschlossen. Die Dichtung wird mittels einer aufblasbaren Dichtungslippe bewerkstelligt. Sie ist am Tor befestigt und schließt in aufgeblasenem Zustand zum seitlichen Umfassung aus Beton hin gasdicht ab. Vor dem Öffnen des Tores wird die Luft aus der Dichtung wieder abgelassen. Der Aufbau dieser Dichtung ist in WO 02/06439 beschrieben.

Die Tore werden nach oben hin geöffnet. Dadurch wird verhindert, dass beim Befüllen oder Entladen ein Radlader versehentlich gegen ein Tor stößt und dieses beschädigt. Die Dichtungslippe ist im Tor befestigt, kann also beim Beladen des Großfermenters ebenfalls nicht verletzt werden.

Die Großfermenter werden mit einem leichten Überdruck von 20 hPa betrieben. Dadurch ist gewährleistet, dass zu keinem Zeitpunkt, auch nicht im Falle einer Leckage, ein explosionsgefährliches Gas-Luft-Gemisch entstehen kann.

Die Bodenplatte 22 der Großfermenter werden aus gasdichtem Beton hergestellt. Die Großfermenter sind langgestreckt garagenartig aufgebaut und können mit Radladern oder Frontladern befahren werden. Mehrere Fermenter werden in einem Block nebeneinander errichtet und zeitlich versetzt betrieben.

Da in den erfindungsgemäßen Trockenfermentationsanlagen Biomassen mit sehr hohem Trockensubstanzgehalt verarbeitet werden, ergibt sich eine kompakte Bauweise der Fermenter und damit der gesamten Anlage. Durch den modulartigen Aufbau mit mehreren Fermentern läßt sich die Anlage ohne großen Aufwand erweitern, wenn zu einem späteren Zeitpunkt die Kapazität erhöht werden soll.

Die Großfermenter eignen sich hervorragend als Ergänzung für bestehende Kompostierungsanlagen. Organische Reststoffe können zur Energiegewinnung genutzt werden, ohne dass die Verarbeitungsschiene auf Flüssigkeiten umgestellt werden muss. Die vorhandenen Gerätschaften können für den Betrieb der Biogasanlage mitgenutzt werden. Die Trockenvergärung kann als zusätzlicher Behandlungsschritt sehr gut in den Betriebsablauf der Kompostierung integriert werden.

Bei der Trockenfermentation können etwa die gleichen Gaserträge erzielt werden wie bei Nassvergärungsverfahren. Hervorzuheben sind der niedrige Schwefelgehalt und der hohe Methangehalt im Biogas bei der Trockenfermentation. Nach bisherigen Erkenntnissen ist keine Entschwefelung des Biogases erforderlich. Das in der Trockenfermentationsanlage gewonnene Biogas wird getrocknet, danach werden Gasqualität und -menge gemessen. Über eine Gasregelstrecke und einen Gasverdichter wird das Biogas dem Blockheizkraftwerk (BHKW) zugeführt. Das BHKW wird entsprechend dem Gasanfall geregelt, so dass keine aufwändige externe Gasspeicherung notwendig ist. Nur der Gasraum über dem Gärsubstrat in den Fermentern und im Falle der Doppelfolie der Gasraum 56 zwischen den beiden Folien 54 und 55 wird als Zwischenspeicher genutzt.

Mit dem BHKW wird das Biogas in Strom und Wärme umgewandelt. Der Strom wird zum gültigen Einspeisetarif am Standort in das Stromnetz eingespeist. Die Wärme wird zu einem geringen Teil zur Beheizung der Anlage verwendet. Der Großteil steht externen Wärmeverbrauchern zur Verfügung.

Neben der Nutzung in einem BHKW bestehen auch andere Möglichkeiten der Biogasnutzung: Nach einer Reinigung zur Anpassung an Erdgasqualität kann Biogas in Erdgasfahrzeugen verwendet oder direkt in das Erdgasnetz eingespeist werden.

Die Trockenfermentationsanlagen gemäß der vorliegenden Erfindung verfügen über ein ausgefeiltes Sicherheitskonzept. Beispielsweise wird der Übergang von der Methanatmosphäre zur Luftatmosphäre vor dem Entleeren der Fermenter ex-geschützt bewerkstelligt. Zu keinem Zeitpunkt kann dabei im Fermenter ein explosionsgefährliches Luft-Methan-Gemisch entstehen. Während dem Befüllen und Entleeren sorgt eine Absaugung im hinteren Bereich des Fermenters dafür, dass dieser ständig von Frischluft durchströmt ist. So ist eine eventuelle Geruchsbelästigung für den Fahrer des Radladers ausgeschlossen. Durch Lichtschranken wird verhindert, dass die Tore versehentlich geschlossen werden, während sich eine Person im Fermenterraum befindet. Zusätzlich ist das Technikgebäude so positioniert, dass vom Steuerungsraum aus der direkte Blick auf die Tore möglich ist.

Obwohl die erfindungsgemäßen Biogasanlagen aufgrund ihrer robusten Technik eine hohe Betriebssicherheit aufweisen, sind zusätzliche Vorkehrungen für den Fall eventueller Betriebsstörungen getroffen. So sind beispielsweise die hydraulisch betriebenen Tore so gesichert, dass sie auch dann nicht herunterfallen können, wenn die Hydraulik nicht funktionsfähig sein sollte. Falls das BHKW einmal ausfällt, kann das Biogas über eine Fackel verbrannt werden.

Die Trockenfermentationsanlage wird über ein rechnergestütztes System gesteuert. Im Perkolatkreislauf, bei der Beheizung und im BHKW-Betrieb können verschiedene Prozessparameter jeweils für die einzelnen Gärbehälter eingestellt werden. Die kontinuierliche Überwachung der Kontrollparameter ermöglicht die ständige Optimierung des Prozesses und damit eine maximale Abbauleistung im Fermenter.

Fig. 8 zeigt eine Variante der Ausführungsform nach Fig. 7, die sich von der Ausführungsform nach Fig. 7 dadurch unterscheidet, dass ein oder mehrere Großfermenter 20 mit Doppelfolie 52 als Hülle 44 vorgesehen werden. Zusätzlich mündet eine zweite Abgasleitung 72 von dem Abgaskühler 64 kommend in den Gasraum 56 der Doppelfolie 52 und aus dem Gasraum 56 heraus führt eine zweite Auspuffleitung 74. Über die zweite Abgasleitung 72 und die zweite Auspuffleitung 74 kann gekühltes Abgas durch den Gasraum 56 der Doppelfolie 52 gepumpt werden. Auf diese Weise der Großfermenter thermisch isoliert werden. Der Grad der Abkühlung des Abgases kann hierbei den Umgebungstemperaturen und der Widerstandsfähigkeit der Doppelfolie 52 angepaßt werden.

Bei allen vorstehend beschriebenen Ausführungsformen der Erfindung mit Doppelfolie 52 kann anstelle von Gas Isoliermaterial, z. B. Steinwolle in den Hohlraum 56 zwischen innerer und äußerer Folie 54, 55 eingebracht werden. Durch diese thermische Isolierung wird der Großfermenter thermische von den Umgebungstemperaturen entkoppelt.

### Bezugszeichenliste

- 2, 50, 60: Großfermenter
- 4: Biogasverbraucher
- 6: Biomasse
- 8: Biogasentnahmeleitung
- 10: Heizleutung zum Großfermenter
- 12: Perkulatsammelleitung
- 14: Perkolatkonzentriereinrichtung
- 16: Perkulatsammeltank
- 17: Perkulatheizleitung
- 18: Perkulatverteilerleitung
- 19: Perkulatverteiler
- 20: Faulbehälter
- 22: Bodenplatte
- 23: Heizelement in der Bodenplatte 22
- 24: stabile Umfassung
- 26, 62: linke Seitenwand der stabilen Umfassung 24
- 27, 63: rechte Seitenwand der stabilen Umfassung 24
- 28: Frontseite des Großfermenters
- 30: Be- und Entladeöffnung
- 32: gasdichte Klappe
- 36: Säulen der stabilen Umfassung
- 38: Zwischenräume in der stabilen Umfassung
- 40: Wandelement der stabilen Umfassung
- 42: Stütz- bzw. Dachgerüst des Großfermenters
- 44: Hülle des Großfermenters
- 46: gasdichte Folie
- 52: Doppelfolie
- 54: innere Folie der Doppelfolie 52
- 55: äußere Folie der Doppelfolie 52
- 56: Gasraum zwischen innerer und äußerer Folie
- 58: Sockel der stabilen Umfassung
- 62: erste Abgasleitung
- 64: Abgaskühler
- 66: Abgasspülleitung
- 68: erste Auspuffleitung
- 70: Ventile
- 72: zweite Abgasleitung
- 74: zweite Auspuffleitung

## Patentansprüche

1. Biogasanlage zur Erzeugung von thermischer, elektrischer oder mechanischer Energie aus Biomasse, mit
wenigstens einem Fermenter zur Erzeugung von Biogas aus Biomasse nach dem Prinzip der Feststoffmethanisierung,
einem Biogasverbraucher (4) zur Erzeugung von thermischer, elektrischer und/oder mechanischer Energie mittels Verbrennung von Biogas,
einer Biogasleitung (8) zur Zuführung des Biogases aus dem wenigstens einen Fermenter (2; 50; 60) zu dem Biogasverbraucher (4),
einer Abgasspüleinrichtung zum Spülen der Fermenter mit Abgas, das bei der Verbrennung von Biogas in dem Biogasverbraucher entsteht, vor dem Entladen der Fermenter, und
einer Abgaskühleinrichtung zur Kühlung des Abgases aus dem Biogasverbraucher vor der Zuführung zu den Fermentern.

2. Biogasanlage nach Anspruch 1, mit
einer Partialdruckmeßeinrichtung zur Erfassung des Sauerstoffpartialdrucks in dem wenigstens einen Fermenter,
einer Abgasspülleitung zur Zuführung von Abgasen aus dem Biogasverbraucher in den wenigstens einen Fermenter,
einer Ventileinrichtung für jeden Fermenter zum Verbinden der Abgasspülleitung mit dem jeweiligen Fermenter und zum Verbinden des jeweiligen Fermenters mit der Umgebung, und
einer Steuereinrichtung zum Betätigen der Ventileinrichtung und zum Fluten des jeweiligen Fermenters, wenn der Sauerstoffpartialdruck in dem jeweiligen Fermenter einen bestimmten Wert überschreitet.

3. Biogasanlage nach Anspruch 1 oder 2, **dadurch gekennzeichnet, dass** die Abgasspüleinrichtung (62, 64, 66, 68, 70) eine Abgasspülleitung (66) umfaßt, die von dem Abgaskühler (64) zu dem jeweiligen Fermenter (2; 20; 60) führt.

4. Biogasanlage nach einem der vorhergehenden Ansprüche, **gekennzeichnet durch** eine Perkolatkonzentriereinrichtung (14) zum Aufkonzentrieren des Perkolats.

5. Biogasanlage nach einem der vorhergehenden Ansprüche, **dadurch gekennzeichnet, dass** der Fermenter ein Großfermenter ist, der einen Faulbehälter (20) zur Aufnahme der Biomasse (6) umfasst, wobei der Faulbehälter (20) aufweist:
einen Biogasentnahmeanschluß (8),
eine gasdichte Bodenplatte (22),
eine sich von der Bodenplatte (22) in die Höhe erstreckende stabile Umfassung (24),
eine einen Teil der stabilen Umfassung bildende Be- und Entladeöffnung (30) zum Be- und Entladen des Faulbehälters (20) mit Biomasse (6),
wobei die Be- und Entladeöffnung (30) mittels einer Klappe (32) gasdichten verschließbar ist, und
einer die oben offene Anordnung aus Bodenplatte (22) und stabiler Umfassung (24) gasdicht abschließenden Hülle (44) in Form einer gasdichten Folie (46; 52),
wobei die stabile Umfassung so dimensioniert ist, dass die Biomasse nicht mit der gasdichten Folie (46; 52) in Berührung kommt.

6. Biogasanlage nach Anspruch 5, **dadurch gekennzeichnet, dass** die gasdichte Folie (46; 52) außerhalb der stabilen Umfassung (24) mit der Bodenplatte (22) gasdicht verbunden ist.

7. Biogasanlage nach einem der vorhergehenden Ansprüche 5 bis 6, **dadurch gekennzeichnet, dass** die gasdichte Folie (46; 52) mit der Außenseite der stabilen Umfassung (24) gasdicht verbunden ist.

8. Biogasanlage nach einem der vorhergehenden Ansprüche 5 bis 7, **dadurch gekennzeichnet, dass** die stabile Umfassung eine umlaufende Oberkante aufweist und dass die gasdichte Folie (46; 52) mit der Oberkante gasdicht verbunden ist.

9. Biogasanlage nach einem der vorhergehenden Ansprüche 5 bis 8, **dadurch gekennzeichnet, dass** die gasdichte Folie eine elastischen Folie (46; 52) ist.

10. Biogasanlage nach einem der vorhergehenden Ansprüche 5 bis 9, **dadurch gekennzeichnet, dass** die Hülle (44) aus einer Doppelfolie (52) mit einem Gasraum (56) zwischen den beiden Folien (54, 55) besteht.

11. Biogasanlage nach Anspruch 10, **dadurch gekennzeichnet, dass** das entstehende Abgas über eine erste und eine zweite Abgasleitung (62, 72) in den Gasraum (56) zwischen den beiden Folien (54, 55) der Doppelfolie (52) einbringbar und über eine zweite Auspuffleitung (74) aus dem Gasraum (56) der Doppelfolie (52) abführbar ist.

12. Biogasanlage nach Anspruch 11, **dadurch gekennzeichnet, dass** der Abgaskühler (64) zwischen erste und zweite Abgasleitung (62, 72) geschaltet ist.

13. Biogasanlage nach Anspruch 10, **dadurch gekennzeichnet, dass** in den Gasraum (56) zwischen den beiden Folien (54, 55) Gas zur thermischen Isolierung des Faulbehälters (20) von der Umgebung eingelagert ist.

14. Biogasanlage nach Anspruch 10, **dadurch gekennzeichnet, dass** in den Gasraum (56) zwischen den beiden Folien (54, 55) Isoliermaterial zur thermischen Isolierung des Faulbehälters (20) von der Umgebung eingelagert ist.

15. Biogasanlage nach einem der vorhergehenden Ansprüche 5 bis 14, **dadurch gekennzeichnet, dass** die Be- und Entladeöffnung (30) teilweise durch die Bodenplatte (22) begrenzt ist.

16. Biogasanlage nach einem der vorhergehenden Ansprüche 5 bis 15, **dadurch gekennzeichnet, dass** der Faulbehälter (20) über die Be- und Entladeöffnung (30) befahrbar ist.

17. Biogasanlage nach einem der vorhergehenden Ansprüche 5 bis 16, **dadurch gekennzeichnet, dass** der Faulbehälter quaderförmig nach Art eines Fahrsilos aufgebaut ist.

18. Biogasanlage nach einem der vorhergehenden Ansprüche 5 bis 17, **dadurch gekennzeichnet, dass** über der seitlichen Umfassung ein Stützgerüst (42) vorgesehen ist, auf der die gasdichten Hülle (44) aufliegen kann.

19. Biogasanlage nach einem der vorhergehenden Ansprüche 5 bis 18, **dadurch gekennzeichnet, dass** über der seitlichen Umfassung ein Außengerüst vorgesehen ist, an dem die Hülle befestigt ist.

20. Biogasanlage nach einem der vorhergehenden Ansprüche 5 bis 19, **dadurch gekennzeichnet, dass** über der gasdichten Hülle (44) zumindest teilweise eine widerstandsfähige Schutzhülle vorgesehen ist.

## Claims

1. Biogas plant for generating thermal, electrical or mechanical energy from biomass, comprising
at least one fermenter for generating biogas from biomass based on the principle of methanisation of solid matter,
a biogas consumer (4) for generating thermal, electrical and/or mechanical energy by combusting biogas,
a biogas line (8) for feeding the biogas out of the at least one fermenter (2; 50; 60) to the biogas consumer (4),
a waste gas flushing system for flushing the fermenter with waste gas created during the combustion of biogas in the biogas consumer prior to discharging the fermenters, and
a waste gas cooling device for cooling the waste gas from the biogas consumer prior to feeding it to the fermenters.

2. Biogas plant as claimed in claim 1, comprising
a partial pressure measuring device for detecting the oxygen partial pressure in the at least one fermenter,
a waste gas flushing line for feeding waste gases out of the biogas consumer into the at least one fermenter,
a valve device for each fermenter for connecting the waste gas flushing line to the respective fermenter and for connecting the respective fermenter to the ambient environment, and
a control system for operating the valve device and for flooding the respective fermenter when the oxygen partial pressure in the respective fermenter exceeds a specific value.

3. Biogas plant as claimed in claim 1 or 2, **characterised in that** the waste gas flushing device (62, 64, 66, 68, 70) comprises a waste gas flushing line (66) running from the waste gas cooler (64) to the respective fermenter (2; 20; 60).

4. Biogas plant **characterised by** a percolate concentrating device (14) for concentrating the percolate.

5. Biogas plant as claimed in one of the preceding claims, **characterised in that** the fermenter comprises a large-scale fermenter connected to a digestion tank (20) for accommodating the biomass (6), and the digestion tank (20) has:
a biogas discharge connector (8),
a gas-tight base plate (22),
a rigid surround (24) extending vertically upwards from the base plate (22),
a filling and discharge opening (30) forming a part of the rigid surround for filling and discharging the digestion tank (20) with biomass (6), which filling and discharge opening (30) can be closed in a gas-tight manner by means of a flap (32), and
a cover (44) in the form of a gas-impermeable film (46; 52) closing the upwardly open arrangement of base plate (22) and rigid surround (24) in a gas-tight manner,
and the rigid surround is dimensioned so that the biomass does not come into contact with the gas-impermeable film (46; 52).

6. Biogas plant as claimed in claim 5, **characterised in that** the gas-impermeable film (46; 52) is connected to the base plate (22) in a gas-tight arrangement outside the rigid surround (24).

7. Biogas plant as claimed in one of preceding claims 5 to 6, **characterised in that** the gas-impermeable film (46; 52) is connected to the external face of the rigid surround (24) in a gas-tight manner.

8. Biogas plant as claimed in one of preceding claims 5 to 7, **characterised in that** the rigid surround comprises a circumferentially extending top edge and the gas-impermeable film (46; 52) is connected to the top edge in a gas-tight arrangement.

9. Biogas plant as claimed in one of preceding claims 5 to 8, **characterised in that** the gas-impermeable film is an elastic film (46; 52).

10. Biogas plant as claimed in one of preceding claims 5 to 9, **characterised in that** the cover (44) consists of a double film (52) with a gas chamber (56) between the two films (54, 55).

11. Biogas plant as claimed in claim 10, **characterised in that** the generated waste gas can be introduced into the gas chamber between the two films (54, 55) of the double film (52)via a first and a second waste gas line (62, 72) and can be fed out of the gas chamber (56) of the double film (52) via a second exhaust line (74).

12. Biogas plant as claimed in claim 11, **characterised in that** the waste gas cooler (64) is connected between the first and second waste gas line (62, 72).

13. Biogas plant as claimed in claim 10, **characterised in that** gas is stored in the gas chamber (56) between the two films (54, 55) as a means of thermally insulating the digestion tank (20) from the ambient environment.

14. Biogas plant as claimed in claim 10, **characterised in that** insulating material is incorporated in the gas chamber (56) between the two films (54, 55) as a means of thermally insulating the digestion tank (20) from the ambient environment.

15. Biogas plant as claimed in one of preceding claims 5 to 14, **characterised in that** the filling and discharge opening (30) is partially bounded by the base plate (22).

16. Biogas plant as claimed in one of preceding claims 5 to 15, **characterised in that** the digestion tank (20) can be walked on above the filling and discharge opening (30).

17. Biogas plant as claimed in one of preceding claims 5 to 16, **characterised in that** the digestion tank is erected in a square shape in the manner of a portable silo.

18. Biogas plant as claimed in one of preceding claims 5 to 17, **characterised in that** a support frame (42) is provided above the lateral surround, on which the gas-impermeable cover (44) can lie.

19. Biogas plant as claimed in one of preceding claims 5 to 18, **characterised in that** an outer frame is provided above the lateral surround, to which the cover can be attached.

20. Biogas plant as claimed in one of preceding claims 5 to 19, **characterised in that** a resistant protective cover is provided at least partially above the gas-impermeable cover (44).

## Revendications

1. Installation de biogaz destinée à générer une énergie thermique, électrique ou mécanique à partir de biomasse, comportant :
au moins un fermenteur destiné à générer du biogaz à partir de la biomasse selon le principe de la méthanisation de matière solide,
un dispositif consommateur de biogaz (4) destiné à générer de l'énergie thermique, électrique et/ou mécanique au moyen de la combustion du biogaz,
une conduite de biogaz (8) permettant d'amener le biogaz provenant du fermenteur (2 ; 50 ; 60), au moins au nombre de un, vers le dispositif consommateur de biogaz (4),
un dispositif de rinçage par effluents gazeux destiné à rincer le fermenteur avec les effluents gazeux qui se forment lors de la combustion du biogaz dans le dispositif consommateur de biogaz, avant le déchargement du fermenteur, et
un dispositif de refroidissement des effluents gazeux destiné à refroidir les effluents gazeux issus du dispositif consommateur de biogaz avant leur amenée vers les fermenteurs.

2. Installation de biogaz selon la revendication 1, comportant :
un dispositif de mesure de pression partielle permettant d'enregistrer la pression partielle de l'oxygène dans le fermenteur, au moins au nombre de un,
une conduite de rinçage par effluents gazeux destinée à amener les effluents gazeux provenant du dispositif consommateur de biogaz dans le fermenteur,
au moins au nombre de un,
un dispositif à soupape pour chaque fermenteur, destiné à relier la conduite de rinçage par effluents gazeux au fermenteur respectif et à relier chaque fermenteur respectif à l'environnement, et
un dispositif de commande permettant d'actionner le dispositif à soupape et de remplir le fermenteur respectif lorsque la pression partielle de l'oxygène dans le fermenteur respectif a dépassé une valeur déterminée.

3. Installation de biogaz selon la revendication 1 ou 2, **caractérisée en ce que** le dispositif de rinçage par effluents gazeux (62, 64, 66, 68, 70) comporte une conduite de rinçage par effluents gazeux (66) qui mène du refroidisseur d'effluents gazeux (64) à chaque fermenteur (2 ; 20 ; 60).

4. Installation de biogaz selon l'une des revendications précédentes, **caractérisée par** un dispositif concentrateur de percolat (14) destiné à concentrer le percolat.

5. Installation de biogaz selon l'une des revendications précédentes, **caractérisée en ce que** le fermenteur est un fermenteur grande capacité qui comprend un digesteur (20) destiné à recevoir la biomasse (6), le digesteur (20) présentant :
un raccord de déchargement du biogaz (8),
une plaque de fond étanche aux gaz (22),
une enceinte stable (24) qui s'étend en hauteur depuis la plaque de fond (22),
une ouverture de chargement et déchargement (30) formant une partie de l'enceinte stable et destinée à charger et décharger le digesteur (20) en biomasse (6), l'ouverture de chargement et déchargement (30) pouvant être fermée de façon étanche aux gaz par une trappe (32),
une enveloppe (44) fermant de façon étanche aux gaz l'agencement ouvert en partie supérieure de la plaque de fond (22) et de l'enceinte stable (24),
sous la forme d'une feuille étanche aux gaz (46 ; 52),
l'enceinte stable étant dimensionnée de façon telle que la biomasse ne touche pas la feuille étanche aux gaz (46 ; 52).

6. Installation de biogaz selon la revendication 5, **caractérisée en ce que** la feuille étanche aux gaz (46 ; 52) est reliée de façon étanche aux gaz à la plaque de fond (22) à l'extérieur de l'enceinte stable (24).

7. Installation de biogaz selon l'une des revendications 5 à 6, **caractérisée en ce que** la feuille étanche aux gaz (46 ; 52) est reliée de façon étanche aux gaz à la partie extérieure de l'enceinte stable (24).

8. Installation de biogaz selon l'une des revendications 5 à 7, **caractérisée en ce que** l'enceinte stable présente une arête périphérique supérieure et **en ce que** la feuille étanche aux gaz (46 ; 52) est reliée de façon étanche aux gaz à l'arête supérieure.

9. Installation de biogaz selon l'une des revendications 5 à 8, **caractérisée en ce que** la feuille étanche aux gaz est une feuille élastique (46 ; 52).

10. Installation de biogaz selon l'une des revendications 5 à 9, **caractérisée en ce que** l'enveloppe (44) se compose d'une double feuille (52) comportant un espace gazeux (56) entre les deux feuilles (54, 55).

11. Installation de biogaz selon la revendication 10, **caractérisée en ce que** les effluents gazeux formés peuvent être amenés par une première et une seconde conduites d'effluents gazeux (62, 72) dans l'espace gazeux (56) entre les deux feuilles (54, 55) de la double feuille (52) et peuvent être purgés de l'espace gazeux (56) de la double feuille (52) par une seconde conduite d'échappement (74).

12. Installation de biogaz selon la revendication 11, **caractérisée en ce que** le refroidisseur d'effluents gazeux (64) est intercalé entre la première et la seconde conduites d'effluents gazeux (62, 72).

13. Installation de biogaz selon la revendication 10, **caractérisée en ce que** dans l'espace gazeux (56) entre les deux feuilles (54, 55) est introduit du gaz destiné à isoler thermiquement le digesteur (20) de l'environnement.

14. Installation de biogaz selon la revendication 10, **caractérisée en ce que** dans l'espace gazeux (56) entre les deux feuilles (54, 55) est introduit un matériau isolant destiné à isoler thermiquement le digesteur (20) de l'environnement.

15. Installation de biogaz selon l'une des revendications 5 à 14, **caractérisée en ce que** l'ouverture de chargement et de déchargement (30) est délimitée partiellement par la plaque de fond (22).

16. Installation de biogaz selon l'une des revendications 5 à 15, **caractérisée en ce que** le digesteur (20) est visitable par l'ouverture de chargement et de déchargement (30).

17. Installation de biogaz selon l'une des revendications 5 à 16, **caractérisée en ce que** le digesteur est construit en forme de parallélépipède à la façon d'un silo tranché.

18. Installation de biogaz selon l'une des revendications 5 à 17, **caractérisée en ce qu'**est prévue au-dessus de l'enceinte latérale une charpente de support (42) sur laquelle peut reposer l'enveloppe (44) étanche aux gaz.

19. Installation de biogaz selon l'une des revendications 5 à 18, **caractérisée en ce qu'**est prévue au-dessus de l'enceinte latérale une charpente extérieure à laquelle est fixée l'enveloppe.

20. Installation de biogaz selon l'une des revendications 5 à 19, **caractérisée en ce qu'**est prévue au moins partiellement une enveloppe protectrice résistante au-dessus de l'enveloppe étanche aux gaz (44).
